# EUROPEAN PATENT APPLICATION

(11) **EP 1 875 895 A1**
(43) Date of publication of application: **09.01.2008**
(21) Application number: 06291137.5
(22) Date of filing: 07.07.2006
(51) Int. Cl.: A61K 8/73, A61K 8/34, A61K 8/41

(54) **Electrolyte containing compositions having enhanced viscosity**

(71) Applicant: Johnson & Johnson Consumer France SAS, 92787 Issy les Moulineaux Cedex 9 (FR)
(72) Inventor: Brillouet, Anne-Sophie, 27400 Louviers (FR); Gohier, Annie, 27310 Trinité de Thouberville (FR); Moisson, Stéphanie, 27400 Acquingy (FR)
(74) Representative: Metten, Karl-Heinz

(57) **Abstract**

The present invention relates to a composition including (i) at least one gum, (ii) at least one starch, and (iii) at least one electrolyte, wherein the composition includes at least about 2%, by weight, of the at least one electrolyte, and the use thereof for the preparation of a cosmetic article to be used for the topical application to skin, hair or nails..

## Description

### BACKGROUND OF THE INVENTION

Many skin care actives are electrolytes and often are only active at either a high or low pH. However, it can be difficult to obtain the desired viscosity for topical compositions, such as emulsions and gels that contain a high concentration of such electrolytes because the presence of the electrolytes and/or the desired pH can result in poor stability of the composition. The present invention relates to the unexpected discovery that the combination of gums and starches are effective for increasing the viscosity of topical compositions containing high levels of electrolytes. The present invention, thus, allows the inclusion of high levels of electrolyte skincare actives in consumer desirable viscous topical compositions, such as an emulsion and gel.

### SUMMARY OF THE INVENTION

The present invention relates to a composition including (i) at least one gum, (ii) at least one starch, and (iii) at least one electrolyte, wherein the composition includes at least about 2%, by weight, of the at least one electrolyte. The present invention also relates to the use of such a composition for the preparation of a cosmetic article to be used for the topical application to skin, hair or nails.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

### Definitions

As used herein, "topical application" and "topically applying" means directly laying on or spreading on the skin, hair, or nail, e.g., by use of the hands or an applicator such as a wipe.

As used herein, "cosmetically-acceptable" means that cosmetically active agents, inert ingredients, or composition which the term describes are suitable for use in contact with tissues (e.g., the skin) without undue toxicity, incompatibility, instability, irritation, allergic response, and the like, commensurate with a reasonable benefit/risk ratio.

### Gums

The compositions of the present invention include one or more polysaccharide gums. Examples of polysaccharide gums include, but are not limited to, sclerotium gum, xanthan gum, guar gum, carob gum, carrageenan, alginates, pectin, karaya gum, gelatin, agar, arabic gum, cellulose gums, and tragacanth gum, sclerotium gum being preferred.

In one embodiment, the composition comprises a cosmetically-acceptable amount of the one or more polysaccharide gums. The gum(s) typically will be present in the composition in an amount from about 0.001% to about 10% by weight, in particular in an amount from about 0.01% to about 5% by weight, such as from about 0.1% to about 1%, by weight.

### Starches

The compositions of the present invention include one or more starches. In one embodiment, the starch is a chemically modified starch (for example, a natural starch that has been altered by cross-bonding, esterification, or convertion by acids or enzymes). Examples of a modified starches include, but are not limited to, aluminum starches, such as aluminium starch octenylsuccinate, and hydroxyalkyl starches such as hydroxypropyl starch phosphate.

In one embodiment, the composition comprises a cosmetically-acceptable amount of the one or more modified starches. The modified starch(es) typically will be present in the composition in an amount from about 0.01% to about 10% by weight, in particular in an amount from about 0.1% to about 5% by weight.

### Electrolytes

The compositions of the present invention include one or more electrolytes (e.g., compounds that when dissolved form ions, e.g. in water).

In one embodiment, the electrolyte is a base. Examples of bases include, but are not limited to, sodium hydroxide, and hydroxyalkyl substituted amines such as triethanolamine, aminomethylpropandiol, thromethamine, tetrahydroxypropyl ethylenediamine, and dimethylaminoethanol. Most preferred are tetrahydroxypropyl ethylenediamine and dimethylaminoethanol.

In one embodiment, the electrolyte is an acid. Examples of acids include, but are not limited to, hydroxy acids such as alpha-hydroxy acids (e.g., glycolic acid, citric acid, lactic acid, alpha-hydroxyethanoic acid, alpha-hydroxyoctanoic acid, alpha-hydroxycaprylic acid), beta-hydroxy acids (e.g., salicylic acid), inorganic acids such a hydrochloric acid, p-anisic acid, and sorbic acid.

In one embodiment, the electrolyte is a salt. Examples of salts include sodium chloride, calcium chloride, potassium chloride.

In one embodiment, the composition comprises a cosmetically-acceptable amount of the one or more electrolytes. The electrolyte(s) typically will be present in the composition in an amount from about 2% to about 10% by weight, in particular in an amount from about 2% to about 8% by weight.

### Compositions

The compositions useful in the present invention involve formulations suitable for administering to the target tissues, such as mammalian skin such as human skin. In one embodiment, the composition contains a cosmetically-acceptable amount of (i) a gum(s), (ii) a modified starch(es), (iii) electrolyte(s), and (iv) a cosmetically-acceptable carrier. In one embodiment, the cosmetically-acceptable carrier is from about 50% to about 97.989%, by weight, of the composition (e.g., from about 80% to about 95%, by weight, of the composition).

The compositions may be made into a wide variety of cosmetic articles that include but are not limited to lotions, creams, gels, sticks, sprays, ointments, cleansing liquid washes and solid bars, shampoos and hair conditioners, pastes, foams, powders, mousses, shaving creams, wipes, strips, patches, electrically-powered patches, wound dressing and adhesive bandages, hydrogels, film-forming products, facial and skin masks, make-up such as foundations, eye liners, and eye shadows, and the like. These product types may contain several types of cosmetically- acceptable carriers including, but not limited to solutions, suspensions, emulsions such as microemulsions and nanoemulsions, gels, solids and liposomes. The following are non-limitative examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include: propylene glycol, polyethylene glycol (e.g. 200-600), polypropylene glycol (e.g. 425-2025), glycerol, 1,2,4-butanetriol, sorbitol esters, 1,2,6-hexanetriol, ethanol, and mixtures thereof.

A lotion can be made from such a solution. Lotions typically contain from about 1% to about 20% (e.g., from about 5% to about 10%) of an emollient(s) and from about 50% to about 90% (e.g., from about 60% to about 80%) of water. As used herein, "emollients" refer to materials used for the prevention or relief of dryness, as well as for the protection of the skin or hair. Examples of emollients include, but are not limited to, those set forth in the International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "ICI Handbook").

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s). Examples of thickening agents include, but are not limited to, those set forth in the ICI Handbook pp. 1693-1697.

The compositions useful in the present invention can also be formulated as emulsions. If the carrier is an emulsion, from about 1% to about 10% (e.g., from about 2% to about 5%) of the carrier contains an emulsifier(s). Emulsifiers may be nonionic, anionic or cationic. Examples of emulsifiers include, but are not limited to, those set forth in the ICI Handbook, pp.1673-1686.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contain between about 0.1% and 5%, by weight, of such gelling agents.

The compositions of the present invention can also be formulated into a solid formulation (e.g., a wax-based stick, soap bar composition, powder, and wipe containing powder).

The compositions useful in the subject invention may contain, in addition to the aforementioned components, a wide variety of additional oil-soluble materials and/or water-soluble materials conventionally used in compositions for use on skin at their art-established levels.

In one embodiment, the composition has a viscosity of greater than about 500 cps, such as greater than 1000 cps, such as greater than 5000 cps (measured using a Brookfield apparatus (spindle 4) at room temperature).

### Retinoids

In one embodiment, the compositions of the present invention also can contain one or more retinoids. Examples of retinoids include, but are not limited to, retinol, retinal, retinoic acid, etretinate, acitretin, adapalene, tazarotene, and alitretinoin, and salts and esters thereof, such a retinyl palmitate and retinyl acetate, as well as mixtures thereof.

In one embodiment, the composition comprises a cosmetically-acceptable amount of the retinoid. The retinoid typically will be present in the composition in an amount from about 0.001% to about 10% by weight, in particular in an amount from about 0.01% to about 1% by weight.

### Additional Cosmetically Active Agents

In one embodiment, the topical composition further comprises a cosmetically active agent. What is meant by a "cosmetically active agent" is a compound that has a cosmetic or therapeutic effect on the skin, hair, or nails, e.g., lightening agents, darkening agents such as self-tanning agents, anti-acne agents, shine control agents, anti-microbial agents, anti-inflammatory agents, antimycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the cosmetically active agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, coenzyme Q10, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera, feverfew, and soy, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin A, vitamin Bs (such as vitamin B3, vitamin B5, and vitamin B12), vitamin C, vitamin K, and vitamin E, and derivatives thereof.

In one embodiment, the composition also can contain an antioxidant. Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, ascorbic acid, and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherols (e.g., tocopheryl acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

### Other Materials

Various other cosmetically-active agents may also be present in the skin care products. These include, but are not limited to, skin protectants, humectants, and emollients. The skin care products may also comprise chelating agents (e.g., EDTA), preservatives (e.g., parabens), pigments, dyes, opacifiers (e.g., titanium dioxide), and fragrances.

The composition and products containing such compositions of the present invention may be prepared using methodology that is well known by an artisan of ordinary skill.

### Example 1: Viscosity Assays

The viscosity of compositions containing scleratium gum and/or hydroxypropyl starch phosphate, in amounts set forth below in Tables 1 and 2. The compositions of Table 1 further contained a buffer solution of 1.5% citric acid, 1.99% sodium citrate, and qs100% of water. The compositions of Table 2 further contained 2.05% citric acid, 2.5% dimethylaminoethanol (DMAE), and qs100% water. The viscosities were tested using a Brookfield apparatus (spindle 4) at the recited speed setting at room temperature.

**Table 1**

| | Viscosity in cps | | |
|---|---|---|---|
| PERCENT, BY WEIGHT, OF SCLEROTIUM GUM AND HYDROXYPROPYL STARCH PHOSPHATE | SCLEROTIUM GUM ALONE + BUFFER | HYDROXYPROPYL STARCH POSPHATE ALONE + BUFFER | SCLEROTIUM GUM + HYDROXYPROPYL STARCH POSPHATE + BUFFER |
| 0,2% sclerotium gum + 4% Hydroxypropyl Starch Posphate | 10 Speed 60 | 478 Speed 60 | 2131 Speed 60 |
| 0,2% sclerotium gum + 6% Hydroxypropyl Starch Posphate | 10 Speed 60 | 13000 Speed 30 | 34670 Speed 12 |
| 0,5% sclerotium gum + 4% Hydroxypropyl Starch Posphate | 37 Speed 60 | 478 Speed 60 | 25250 Speed 21 |
| 0,5% sclerotium gum + 6% Hydroxypropyl Starch Posphate | 37 Speed 60 | 13000 Speed 30 | 41790 Speed 12 |
| 1% sclerotium gum + 2% Hydroxypropyl Starch Posphate | 210 Speed 60 | 0 Speed 60 | 3140 Speed 60 |
| 1% sclerotium gum + 4% Hydroxypropyl Starch Posphate | 210 Speed 60 | 478 Speed 60 | 73310 Speed 6 |

**Table 2**

| | VISCOSITY IN CPS | | |
|---|---|---|---|
| AMOUNT OF SCLEROTIUM GUM / HYDROXYPROPYL STARCH POSPHATE | SCLEROTIUM GUM ALONE + DMAE | HYDROXYPROPYL STARCH POSPHATE ALONE + DMAE | SCLEROTIUM GUM + HYDROXYPROPYL STARCH POSPHATE + DMAE |
| 0.2% sclerotium gum + 6% Hydroxypropyl Starch Posphate | 0 Speed 60 | 7031 Speed 60 | 16560 Speed 30 |
| 0.5% sclerotium gum + 4% Hydroxypropyl Starch Posphate | 110 Speed 60 | 181 Speed 60 | 6050 Speed 60 |
| 0.5% sclerotium gum + 6% Hydroxypropyl Starch Posphate | 110 Speed 60 | 7031 Speed 60 | 40620 Speed 12 |
| 1% sclerotium gum + 4% Hydroxypropyl Starch Posphate | 1200 Speed 60 | 181 Speed 60 | 34840 Speed 12 |
| 1% sclerotium gum + 6% Hydroxypropyl Starch Posphate | 1200 Speed 60 | 7031 Speed 60 | 41700 Speed 12 |

### Example 2: Topical Composition

Table 3 recites the ingredient list of a topical emulsion of the present invention.

**Table 3**

| INGREDIENT | %(W/W) |
|---|---|
| Water | 56.96 |
| Sclerotium Gum | 0.25 |
| Hydroxypropyl starch phosphate | 3 |
| Glycerin | 10 |
| PEG 8 | 4 |
| Octyl Palmitate | 5 |
| isononylisononanoate | 5 |
| Cethyl Alcohol | 0.75 |
| Stearyl alcohol | 0.75 |
| Cetyl Alcohol & Glyceryl Stearate & Peg 75 Stearate & Ceteth 20 & Steareth 20 | 2 |
| Phenoxyethanol / Methylparaben / Ethylparaben / Butylparaben / Propylparaben | 0.8 |
| Water | 6 |
| Citric Acid | 1.5 |
| Sodium citrate | 1.99 |
| Aluminium Starch Octenylsuccinate | 2 |

The above formulation is made as follows. In a first phase, the water, sclerotium gum, hydroxypropyl starch phosphate, glycerin and PEG 8 are mixed under stirring and heated to 75°C. In a second phase, the octyl palmitate, isononylisononanoate, cethyl alcohol, stearyl alcohol, cetyl alcohol & glyceryl stearate & PEG 75 stearate & ceteth 20 & steareth 20 were mixed under stirring and heated to 75°C. Then the first phase and the second phase were mized under stirring and heated to 75°C. The resulting mixture was then allowed to cool down. During cooling, phenoxyethanol & methylparaben & ethylparaben & butylparaben & propylparaben, the additional water, citric acid, sodium citrate, and aluminium starch octenylsuccinate were added while stirring. The viscocity was 9100 cps (speed 60) with Brookfield apparatus (spindle 4) measured at room temperature.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A composition comprising (i) at least one polysaccharide gum, (ii) at least one starch, and (iii) at least one electrolyte, wherein said composition comprises at least about 2%, by weight, of said at least one electrolyte.

2. A composition of according to claim 1, wherein said composition comprises at least about 0.1%, by weight, of said at least one gum.

3. A composition according to claim 1 or 2, wherein said composition comprises at least about 0.1%, by weight, of sclerotium gum.

4. A composition of any of the preceding claims, wherein said composition comprises at least 1%, by weight, of at least one modified starch.

5. A composition of any of the preceding claims, wherein said composition comprises at least about 1%, by weight, of at least one aluminum starch, in particular aluminum starch octenylsuccinate, and/or at least one hydroxyalkyl starch, in particular hydroxypropyl starch phosphate.

6. A composition of any of the preceding claims, wherein said at least one electrolyte is a base selected form the group consisting of tetrahydroxypropyl ethylenediamine and dimethylaminoethanol.

7. A composition of claim 6 wherein said compositon comprises at least 2%, by weight, of tetrahydroxypropyl ethylenediamine, dimethylaminoethanol, or a mixture thereof.

8. A composition of any of the preceding claims, wherein said composition has a viscosity of greater than about 500 cps measured using a Brookfield apparatus (spindle 4) at room temperature.

9. A composition of any of the preceding claims, wherein said composition is in the form of an emulsion.

10. A composition of any of the preceding claims, wherein said composition further comprises one or more retinoids.

11. Use of a composition according to anyone of the preceding claims for the preparation of a cosmetic article to be used for the topical application to skin, hair or nails.
